# EUROPÄISCHE PATENTSCHRIFT

(11) **EP 2 349 239 B1**
(45) Veröffentlichungstag und Bekanntmachung des Hinweises auf die Patenterteilung: **15.11.2017**
(21) Anmeldenummer: 09782234.0
(22) Anmeldetag: 26.08.2009
(51) Int. Cl.: A61K 31/357, A61K 31/443, A61P 13/02, A61P 35/00, A61P 29/00, A61G 9/00, A61K 6/00, A61K 8/42, A61K 8/49, A61Q 5/02, A61Q 11/00, A61K 8/69, A61Q 17/04, A61Q 17/02, A23L 27/00

(54) **NACHWEIS UND VERWENDUNG NIEDERMOLEKULARER MODULATOREN DES KÄLTE-MENTHOL-REZEPTORS TRPM8**
DETECTION AND USE OF LOW MOLECULAR-WEIGHT MODULATORS OF THE COLD-MENTHOL RECEPTOR TRPM8
DÉTECTION ET UTILISATION DE MODULATEURS À FAIBLE POIDS MOLÉCULAIRE DU RÉCEPTEUR AU FROID ET AU MENTHOL TRPM8

(30) Priorität: 26.08.2008 EP 08162997
(43) Veröffentlichungstag der Anmeldung: 03.08.2011
(60) Teilanmeldung: 16175302.5
(73) Patentinhaber: BASF SE, 67056 Ludwigshafen am Rhein (DE)
(72) Erfinder: SUBKOWSKI, Thomas, 68526 Ladenburg (DE); BOLLSCHWEILER, Claus, 69118 Heidelberg (DE); WITTENBERG, Jens, 67117 Limburgerhof (DE); KROHN, Michael, 64653 Lorsch (DE); ZINKE, Holger, 64673 Zwingenberg (DE)
(86) Internationale Anmeldenummer: PCT/EP2009/061019
(87) Internationale Veröffentlichungsnummer: WO 2010/026094

(56) Entgegenhaltungen:
- WO-A-2004/026840
- WO-A-2007/017093
- WO-A-2008/015403
- WO-A1-2005/099711
- WO-A2-2009/064388
- SU-A1- 776 048
- US-A1- 2005 084 447
- US-A1- 2006 167 263
- DATABASE CA [Online] CHEMICAL ABSTRACTS SERVICE, COLUMBUS, OHIO, US 21 September 2004 CHEMICAL LIBRARY SUPPLIER: ENAMINE Retrieved from stn, accession no. 748783-13-3 Database accession no. 748783-13-3
- DATABASE CA [Online] CHEMICAL ABSTRACTS SERVICE, COLUMBUS, OHIO, US 02 April 2006 CHEMICAL LIBRARY SUPPLIER: ENAMINE Retrieved from STN, accession no. 878942-21-3 Database accession no. 878942-21-3

## Beschreibung

Die Erfindung betrifft neuartige Modulatoren des Kälte-Menthol-Rezeptors TRPM8, Verfahren zur Modulation des TRPM8-Rezeptors unter Verwendung dieser Modulatoren; die Verwendung der Modulatoren zur Induktion von Kältegefühl; sowie die unter Verwendung dieser Modulatoren hergestellten Gegenstände und Mittel.

### Hintergrund der Erfindung

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP channel that senses cold stimuli and menthol.Cell. 2002 Mar 8;108(5):705-15; McKemy DD et al. Identification of a cold receptor reveals a general role for TRP channels in thermosensation. Nature. 2002 Mar 7;416(6876):52-8; Zuker CS. Neurobiology: a cool ion channel.Nature. 2002 Mar 7;416(6876):27-8)

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005,16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971#1315761; Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8 Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8 Rezeptor bzw. seine Insektenanaloga modulieren, sind eine Repellentwirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8 Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

### Kurzfassung der Erfindung

Aufgabe der vorliegenden Erfindung war es daher, neue Substanzen zu identifizieren, die zu einer Modulation des TRPM8 Rezeptors führen, welche als Alternativen zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche), Verpackungen oder als Insektizid bzw. Repellent eignen.

### Figurenbeschreibung:

Figur 1 zeigt (a) die mRNA-Sequenz bzw. (b) die davon abgeleitete Aminosäuresequenz des hTRPM8 Rezeptors gemäß Sequenzdatenbank-Eintrag NM 024080.
Figur 2 zeigt die Vektorkarte des mit hTRPM8 kodierenden Plasmids plnd_M8, welches zur Transfektion der HEK293 Zellen verwendet wurde.

### Detaillierte Beschreibung der Erfindung:

### 1. Definition allgemeiner Begriffe:

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist und/oder Antagonist des TRPM8-Rezeptors in vivo und/oder in vitro wirken kann.

Geeignete Modulatoren können dabei entweder nur als Antagonist oder Agonist oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt. Ein "Antagonist" ist dagegen eine Verbindung, welche dieser Aktivierung des TRPM8-Rezeptors entgegenwirken kann.

Die erfindungsgemäßen Mediatoren können ihre Wirkung dadurch ausüben, dass sie reversibel oder irreversibel, spezifisch oder unspezifisch an ein TRPM8 Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8 Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische und/oder antagonistische Modulation (wie oben beschrieben) des TRPM8 Rezeptors feststellen lässt.

### 2. Bevorzugte Ausführungsformen

Ein erster Gegenstand der Erfindung betrifft ein Verfahren zur in-vitro Modulation des Kälte-Menthol-Rezeptors TRPM8, insbesondere des humanen TRPM8 Rezeptors, wobei man den Rezeptor mit wenigstens einer Verbindung in Kontakt bringt, die ausgewählt ist unter Verbindungen der Formeln 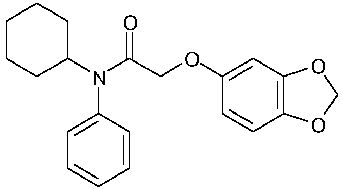 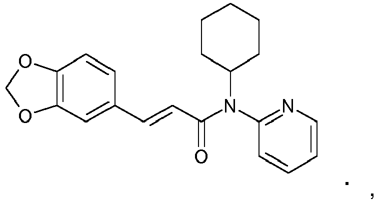 welche in einem zellulären Aktivitätstest, insbesondere unter Standardbedingungen, unter Verwendung von Zellen, welche den humanen TRPM8-Rezeptor rekombinant exprimieren, die Permeabilität dieser Zellen für Ca²⁺ Ionen modulieren.

Unter "Standardbedingungen" versteht man in diesem Zusammenhang einen Aktivitätstest, durchgeführt mit HEK293-Zellen, welche transformiert wurden mit humanem TRPM8 und beladen sind mit Calcium-sensitivem Farbstoff (wie z.B. Fluo-4AM, d.h. Fluo-4-Acetoxymethylester), anschließende Zugabe der Testverbindung und Nachweis der Farbänderung, wobei Versuchsdurchführung bei 37°C erfolgt; wie z.B. beschrieben in Beispiel 3, unten, oder bei Behrendt et al (2004) a.a.O).

Die erfindungsgemäß eingesetzten Modulatoren wirken dabei auf die zelluläre Ca²⁺-Ionen-Permeabilität agonistisch oder antagonistisch. Insbesondere ist der Modulator wenigstens eine Verbindung, ausgewählt unter Verbindungen der folgenden Formeln 4 und 5 gemäß folgender Tabelle 1 (die Verbindungen der Formeln 1-3 sowie 6-19 sind Referenzbeispiele),

**Tabelle 1: Erfindungsgemäße Modulatoren und Referenzbeispiele**

| | |
|---|---|
| 1 | 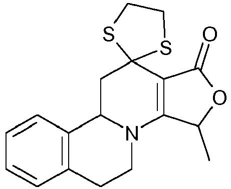 |
| 2 | 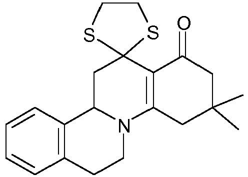 |
| 3 | 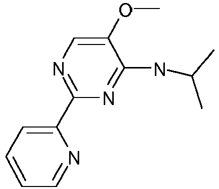 |
| 4 | 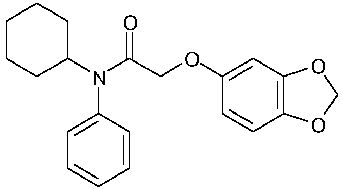 |
| 5 | 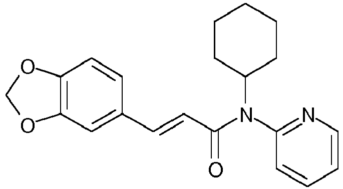 |
| 6 | 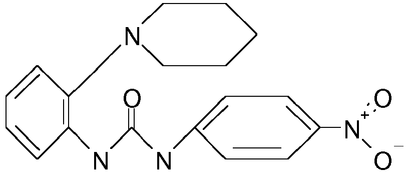 |
| 7 | 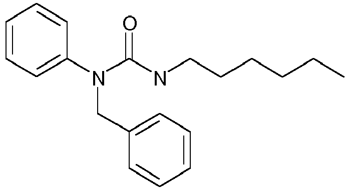 |
| 8 | 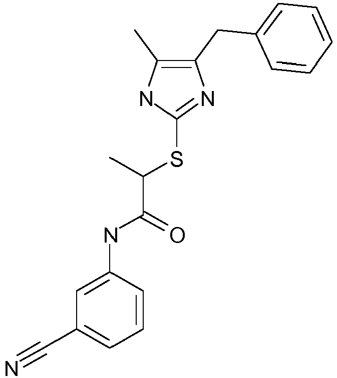 |
| 9 | 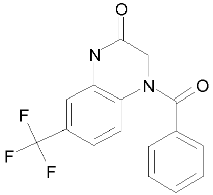 |
| 10 | 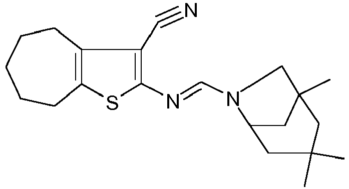 |
| 11 | 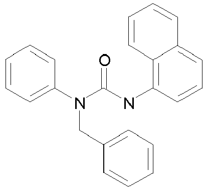 |
| 12 | 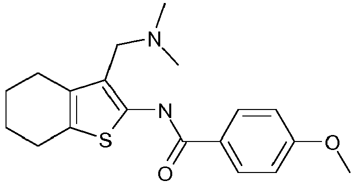 |
| 13 | 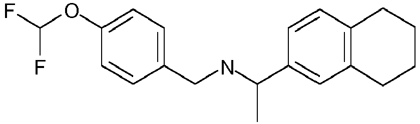 |
| 14 | 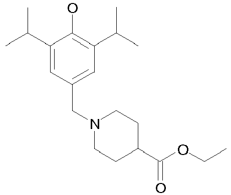 |
| 15 | 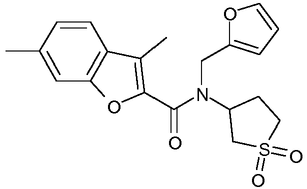 |
| 16 | 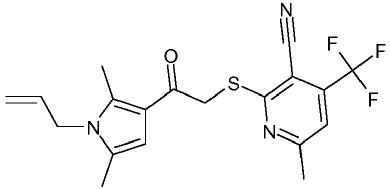 |
| 17 | 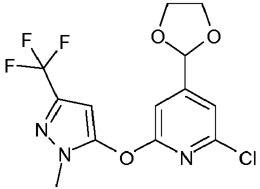 |
| 18 | 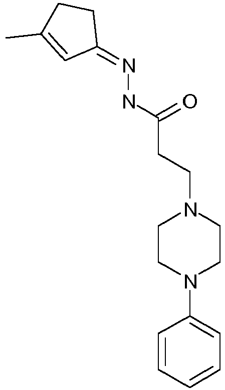 |
| 19 | 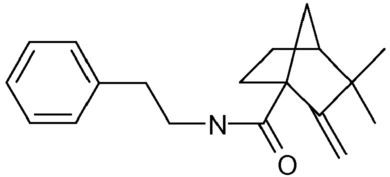 |

wobei die Verbindung in chemisch reiner oder angereicherter Form, als einzelnes Stereoisomer oder in Form von Stereoisomeren-Gemischen vorliegen kann. Weiterhin können die Verbindungen ungeladen oder in Form ihrer Salze, wie z.B. als Säureadditionssalz, vorliegen. Funktionelle Gruppen können ggf. durch äquivalente chemische Gruppen ersetzt sein; Fluoratome können so z.B. durch andere Halogenatome, wie Cl, Br oder J, ersetzt sein, Sauerstoffgruppen (wie z.B. Ethergruppen) können durch entsprechende Schwefelgruppen ersetzt sein und umgekehrt; Ketogruppen können durch entsprechende Thionylgruppen ersetzt sein. Die oben genannten Verbindungen sind an sich bekannte chemische Substanzen, die entweder käuflich erhältlich oder unter Anwendung üblicher Methoden der organischen Synthese zugänglich sind.

So sind beispielsweise bekannt:
Verbindung 1 unter CAS Nummer: 99602-94-5 (3R-cis-Form)
Verbindung 2 unter CAS Nummer: 165753-08-2
Verbindung 3 unter CAS Nummer: 338771-57-6
Verbindung 4 unter CAS Nummer: 878942-21-3
Verbindung 5 unter CAS Nummer: 748783-13-3

Die abgewandelten Formen oder Derivate werden auch als funktionale Analoga oder funktional äquivalente Verbindungen bezeichnet, wenn sie weiterhin die gewünschte biologische Aktivität (Rezeptor TRPM8 Modulation) zeigen.

Weiterhin sind besonders Derivate im Sinne der Erfindung mit umfasst, die eine Kopplung der konkret offenbarten Substanzen an feste Träger erlauben; eine große Auswahl an entsprechenden Linkern/Spacer-Gruppen ist dem Fachmann bekannt. Die Derivatisierung kann dabei vor der Kopplung an eine feste Phase oder erst durch die Kopplung erfolgen.

Ein weiterer Gegenstand der Erfindung betrifft die Verwendung eines Modulators, insbesondere Agonisten, für den TRPM8-Rezeptor, wobei der Modulator wie oben definiert ist, zur Induktion von Kältegefühl, insbesondere topisch, d.h. cutan oder oral, bei Mensch und/oder Tier. Eine "Induktion von Kältegefühl" liegt vor, wenn die Verbindung im oben beschriebenen zellulären Aktivitätstest einen agonistischen Effekt auf hTRPM8 zeigt.

Ein weiterer Gegenstand der Erfindung betrifft die Verwendung eines Modulators für den TRPM8-Rezeptor, wobei der Modulator wie oben definiert ist, als aktiven Bestandteil eines pharmazeutischen Mittels.

Ein weiterer Gegenstand der Erfindung betrifft die Verwendung eines Modulators für den TRPM8-Rezeptor, wobei der Modulator wie oben definiert ist, zur Behandlung von Prostatakarzinomen, zur Behandlung von Blasenschwäche oder in der Schmerztherapie.

Ein weiterer Gegenstand der Erfindung betrifft die Verwendung eines Modulators für den TRPM8-Rezeptor, wobei der Modulator wie oben definiert ist, als Insekten-Repellent oder Insektizid.

Ein weiterer Gegenstand der Erfindung betrifft die Verwendung eines Modulators für den TRPM8-Rezeptor, wobei der Modulator wie oben definiert ist, zur Induktion eines Kältegefühls bei Verpackungen (z.B. aus Papier oder Kunststoff in verschiedensten Verarbeitungsformen (wie z.B. Fasern, Geweben, Formteilen), wobei sich das Kältegefühl insbesondere bei Kontakt mit dem Verpackungsmaterial bemerkbar macht. Dabei können die Substanzen in unterschiedlichster Art mit dem Verpackungsmaterial assoziiert sein: z.B. durch Spincoating, Aufdrucken, in Form von Mikroverkapselung, direkte Einarbeitung in das Verpackungsmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft die Verwendung eines Modulators für den TRPM8-Rezeptor, wobei der Modulator wie oben definiert ist, zur Induktion eines Kältegefühls bei Textilien. Dabei können die Substanzen in unterschiedlichster Art mit dem Textil assoziiert sein: z.B. durch Spincoating, Aufdrucken, in Form von Mikroverkapselung, direkte Einarbeitung in das Textilmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.

Ein weiterer Gegenstand der Erfindung betrifft Stoffe per se gemäß obiger Definition zur Verwendung als Mediator, insbesondere Agonisten und/oder Antagonisten, des TRPM8 Rezeptors in einem Medikament.
Ein weiterer Gegenstand der Erfindung betrifft Mittel, enthaltend wenigstens eine Verbindung nach obiger Definition. Insbesondere sind solche Mittel ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi, Atemerfrischungsmittel
d) Haut-, oder Haarpflegemitteln, wie Sonnencreme, Sonnenbrandcreme, Lotionen, Shampoos, Pflaster, Mundwasser, Lotionen, Rasiercreme, Conditioner, Gesichtsreiniger, Seifen, Badeöle und Badeschäume, Antitranspirationsmittel, desodorierende Mittel,
e) Insektenrepellents, Insektiziden.

Derartige Mittel enthalten neben für das jeweilige Mittel jeweils üblichen Bestandteilen eine wirksame Menge wenigstens eines erfindungsgemäßen Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt, oder sensorischen Effekt wie z.B. den olfaktorischen Kälteeffekt zu vermitteln.

Gegebenenfalls können die erfindungsgemäßen Verbindungen mit weiteren bekannten Wirkstoffen, insbesondere auch solchen mit vergleichbarer Wirkung, kombiniert werden. Beispielsweise können diese mit bekannten kühlenden Verbindungen, wie z.B. Menthol, Menthon, N-Ethyl-p-menthancarboxamid (WS-3), N-2,3-Trimethyl-2-isopropylbutanamid (WS-23), Menthyllactat (Frescolat® ML), Menthonglycerinacetal (Frescolat® MGA), Mono-menthylsuccinat (Physcool®), Mono-menthyl glutarat, O-Menthyl Glycerin, Menthyl-N,N-dimethylsuccinamat kombiniert werden.

Gegenstand der Erfindung sind weiterhin Textilprodukte, wie z.B. Hemden, Hosen, Socken, Handtücher, ausgerüstet (insbesondere oberflächlich) mit wenigstens einer Verbindung nach obiger Definition.

Gegenstand der Erfindung sind weiterhin Verpackungsmaterialien, welche mit wenigstens einer Verbindung nach obiger Definition assoziiert sind.

Die Erfindung wird nun anhand folgender nicht-limitierender Ausführungsbeispiele beschrieben.

### Experimenteller Teil:

### Beispiel 1 - Klonierung von humanem TRPM8

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

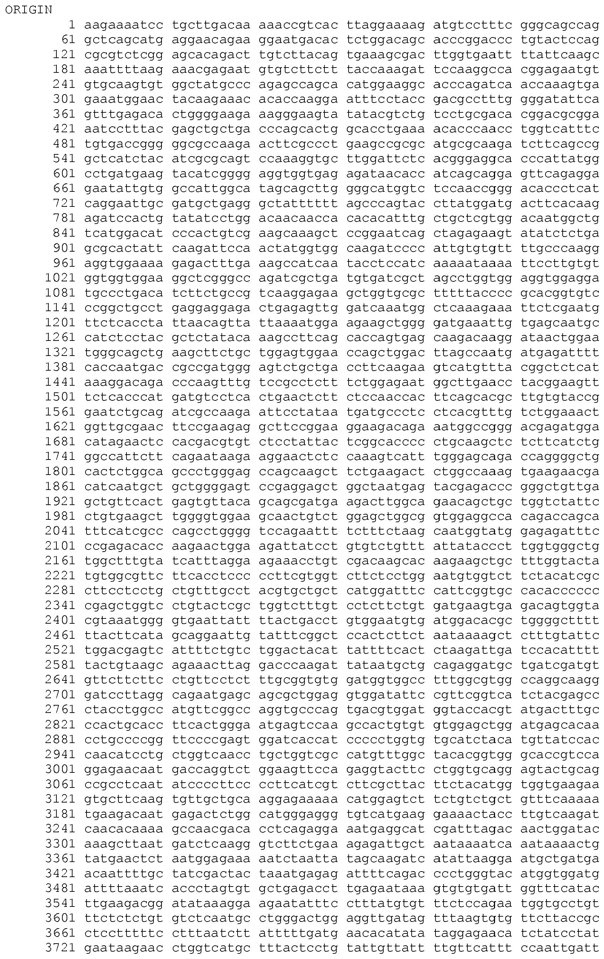

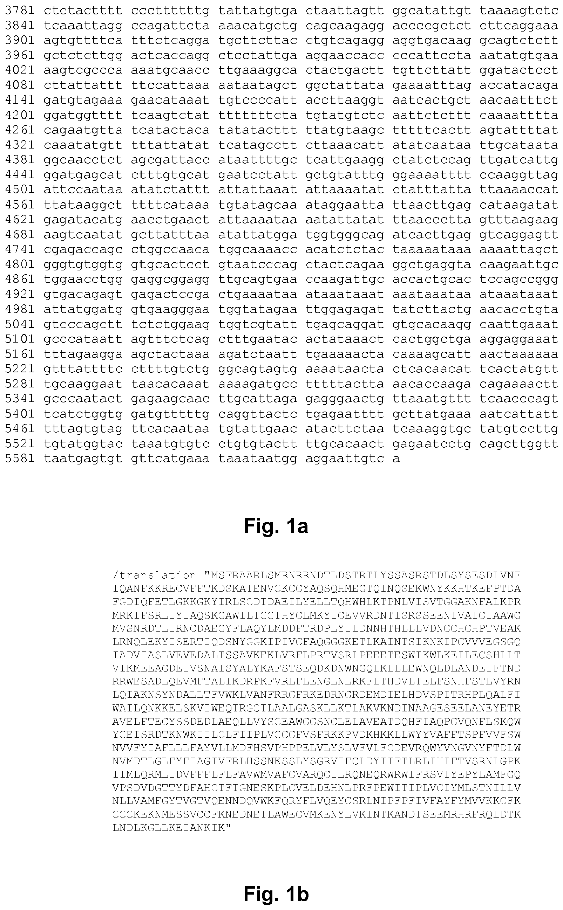

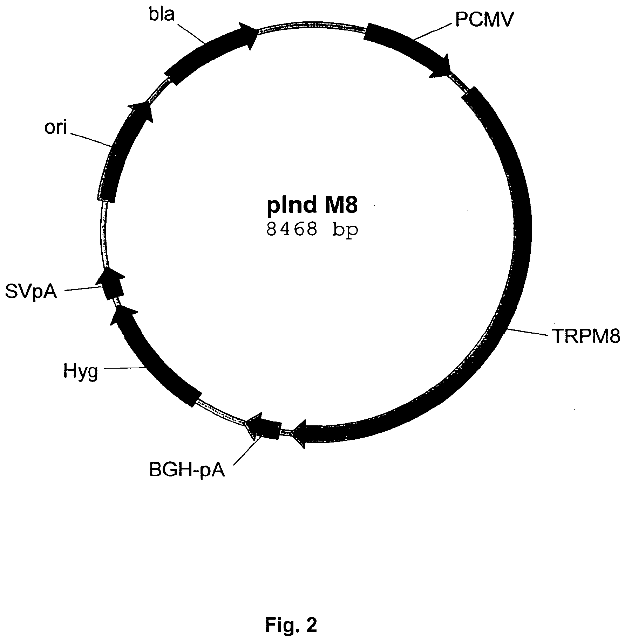

Die kodierende TRPM8 Sequenz (vgl Fig.1A; sowie **http://www.ncbi.nlm.nih.gov/entrez/viewer.fcgi?db=nuccore&id=109689694)** kann unter Verwendung von Standardmethoden PCR-amplifiziert und kloniert werden. Das so isolierte humane TRPM8 Gen wurde zur Herstellung des Plasmids plnd_M8 verwendet, dessen Konstruktion durch die Plasmidkarte gemäß Figur 2 veranschaulicht wird.

Alternativ dazu kann das TRPM8 Gen auch synthetisch hergestellt werden.

### Beispiel 2 - Generierung der HEK293-Testzellen

Als Testzellsystem wurde mit der humanen TRPM8 DNA (vgl obiges Plasmid plnd-M8) stabil transfizierte HEK293 Zelllinie hergestellt. Bevorzugt wird dabei HEK293 die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der erfindungsgemäß verwendeten Zellen den Angaben in Behrendt H.J. et al." Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", zugänglich unter http://www-brs.ub.ruhr-uni-bochum.de/netahtml/HSS/Diss/BehrendtHansJoerg/diss.pdf entnehmen. Auf die Offenbarung dieser Druckschriften wird ausdrücklich Bezug genommen.

### Beispiel 3- Assay auf TRPM8 Modulatoren

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

### a) Testablauf:

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. Ionomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

### b) Testergebnis

Die ermittelten EC50-Werte erfindungsgemäßer Modulatoren sind in folgender Tabelle 2 zusammengefasst

**Tabelle 2: Aktivität von Testsubstanzen auf den human Rezeptor TRPM8**

| **#** | **Activität TRPM8 EC50** |
|---|---|
| 1 | 0,4 |
| 2 | 2 |
| 3 | 2 |
| 4 | 2,5 |
| 5 | 2,5 |
| 6 | 3,5 |
| 7 | 4 |
| 8 | 5 |
| 9 | 10 |
| 10 | 10 |
| 11 | 10 |
| 12 | 10 |
| 13 | 10 |
| 14 | 20 |
| 15 | 20 |
| 16 | 25 |
| 17 | 50 |
| 18 | 100 |
| 19 | 100 |

Die Auswertung zeigt überraschenderweise, dass erfindungsgemäß erstmalig Agonisten von TRPM8 bereitgestellt werden konnten, welche sich strukturell von bisher bekannten Agonisten, wie (-) Menthol, Icilin und anderen von Behrendt H.J. et al.,in Br. J. Pharmacol. 141, 2004, 737-745 (vgl dortige Tabelle 1) beschriebenen Modulatoren signifikant unterscheiden und zudem teilweise bessere Aktivitäten als (-) Menthol zeigen, bzw. vergleichbar stark wirken wie Icilin.

### Beispiel 4 - Herstellung von Mundwasser

Ein Mundwasser folgender Zusammensetzung wird hergestellt:

| | |
|---|---|
| Ethanol 95% | 177mL |
| Sorbit 70% | 250 g |

TRPM8 Agonist gemäß von Tab.2

| | |
|---|---|
| als 1 % Lösung im Ethanol | 50mL |
| Pfefferminzöl, | 0.30 g |
| Methylsalicylat | 0.64 g |
| Eucalyptol | 0.922 g |
| Thymol | 0.639 g |
| Benzoesäure | 1.50 g |

### Pluronic ® F127

| | |
|---|---|
| nichtionisches Tensid | 5.00 g |
| Natrium-Saccharin | 0.60 g |
| Natriumcitrat | 0.30 g |
| Zitronensäure | 0.10 g |

### Wasser q.s. 1 Liter

Zur Herstellung eines Mundwassers werden die oben beschriebenen Komponenten in den angegebenen Mengen miteinander vermischt.

Auf die Offenbarung der hierin zitierten Literaturstellen wird hiermit ausdrücklich Bezug genommen.

## Patentansprüche

1. Verfahren zur in-vitro Modulation des Kälte-Menthol-Rezeptors TRMP8, wobei man den Rezeptor mit wenigstens einer Verbindung in Kontakt bringt, die ausgewählt ist unter den Verbindungen der Formeln 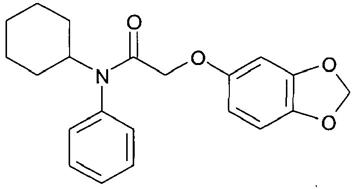 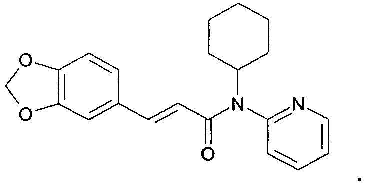 welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

2. Verfahren nach Anspruch 1, wobei der Modulator auf die zelluläre Ca²⁺-Ionen-Permeabilität agonistisch oder antagonistisch wirkt.

3. Verwendung eines Modulators für den TRPM8-Rezeptor, wobei der Modulator gemäß Anspruch 1 definiert ist, zur Induktion von Kältegefühl bei Mensch und/oder Tier.

4. Verwendung eines Modulators für den TRPM8-Rezeptor, wobei der Modulator gemäß Anspruch 1 definiert ist, als aktiven Bestandteil eines pharmazeutischen Mittels.

5. Modulator für den TRPM8-Rezeptor, wobei der Modulator gemäß Anspruch 1 definiert ist, zur Verwendung bei der Behandlung von Prostatakarzinomen, zur Behandlung von Blasenschwäche oder in der Schmerztherapie.

6. Verwendung eines Modulators für den TRPM8-Rezeptor, wobei der Modulator gemäß Anspruch 1 definiert ist, als Insekten-Repellent oder Insektizid.

7. Verwendung eines Modulators für den TRPM8-Rezeptor, wobei der Modulator gemäß Anspruch 1 definiert ist, zur Induktion eines Kältegefühls in einer Verpackung oder einem Textil.

8. Stoff gemäß der Definition nach Anspruch 1 zur Verwendung als Medikament.

9. Mittel enthaltend im Gemisch mit üblichen Bestandteilen wenigstens eine Verbindung nach Anspruch 1.

10. Mittel nach Anspruch 9, ausgewählt unter
a) pharmazeutischen Mitteln, wie Antitumormittel, Mittel zur Behandlung von Erkrankungen der Blase, Schmerzmittel;
b) Nahrungsmitteln, wie Eis, Mousse, Creme, Getränke, Süßwaren,
c) Mundpflegemitteln, wie Zahnpasta, Mundwasser, Kaugummi,
d) Haut- oder Haarpflegemitteln, wie Sonnencreme, Sonnenbrandcreme, Lotionen, Shampoos, Pflaster,
e) Insektenrepellents, Insektiziden.

11. Textilprodukte, wie Hemden, Hosen, Socken, Handtücher, ausgerüstet mit wenigstens einer Verbindung nach Anspruch 1.

12. Verpackungsmaterial, welches mit wenigstens einer Verbindung nach Anspruch 1 assoziiert ist.

## Claims

1. A method for the in-vitro modulation of the cold menthol receptor TRMP8, where the receptor is brought into contact with at least one compound which is selected from the compounds of the formulae 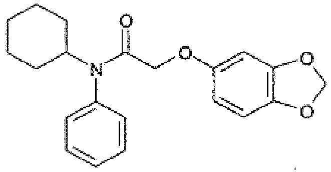 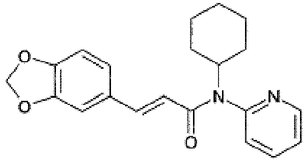 which, in a cellular activity test using cells which recombinantly express the human TRPM8 receptor, modulate the permeability of these cells for Ca²⁺ ions.

2. The method according to claim 1, where the modulator has an agonistic or antagonistic effect on the cellular Ca²⁺ ion permeability.

3. The use of a modulator for the TRPM8 receptor, where the modulator is defined according to claim 1, for inducing a sensation of coldness in humans and/or animals.

4. The use of a modulator for the TRPM8 receptor, where the modulator is defined according to claim 1, as active constituent of a pharmaceutical composition.

5. A modulator for the TRPM8 receptor, where the modulator is defined according to claim 1, for use in the treatment of prostate carcinomas, for the treatment of bladder weakness or in pain therapy.

6. The use of a modulator for the TRPM8 receptor, where the modulator is defined according to claim 1, as insect repellent or insecticide.

7. The use of a modulator for the TRPM8 receptor, where the modulator is defined according to claim 1, for inducing a sensation of coldness in a packaging or a textile.

8. A substance according to the definition according to claim 1 for use as medicament.

9. A composition comprising, in a mixture with customary constituents, at least one compound according to claim 1.

10. The composition according to claim 9, selected from
a) pharmaceutical compositions, such as antitumor agents, agents for the treatment of diseases of the bladder, painkillers;
b) foods, such as ice cream, mousse, cream, beverages, confectionery,
c) mouthcare compositions, such as toothpaste, mouthwash, chewing gum,
d) skincare or haircare compositions, such as suncream, sunburn cream, lotions, shampoos, plasters,
e) insect repellents, insecticides.

11. A textile product such as a shirt, trousers, socks, a towel, finished with at least one compound according to claim 1.

12. A packaging material which is associated with at least one compound according to claim 1.

## Revendications

1. Procédé de modulation in vitro du récepteur au froid et au menthol TRMP8, selon lequel le récepteur est mis en contact avec au moins un composé qui est choisi parmi les composés des formules 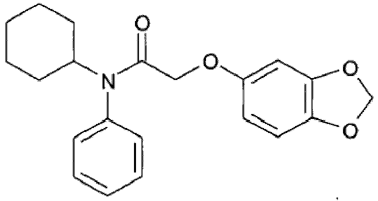 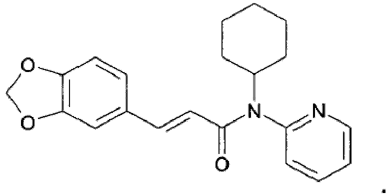 qui, lors d'un test d'activité cellulaire utilisant des cellules qui expriment de manière recombinante le récepteur TRPM8 humain, modulent la perméabilité de ces cellules pour les ions Ca²⁺.

2. Procédé selon la revendication 1, selon lequel le modulateur a un effet agonistique ou antagonistique sur la perméabilité cellulaire pour les ions Ca²⁺.

3. Utilisation d'un modulateur pour le récepteur TRPM8, le modulateur étant défini selon la revendication 1, pour l'induction d'une sensation de froid chez un homme et/ou un animal.

4. Utilisation d'un modulateur pour le récepteur TRPM8, le modulateur étant défini selon la revendication 1, en tant que constituant actif d'un agent pharmaceutique.

5. Modulateur pour le récepteur TRPM8, le modulateur étant défini selon la revendication 1, destiné à une utilisation lors du traitement de cancers de la prostate, pour le traitement d'une faiblesse de la vessie ou dans le traitement de la douleur.

6. Utilisation d'un modulateur pour le récepteur TRPM8, le modulateur étant défini selon la revendication 1, en tant qu'agent répulsif contre les insectes ou insecticide.

7. Utilisation d'un modulateur pour le récepteur TRPM8, le modulateur étant défini selon la revendication 1, pour l'induction d'une sensation de froid dans un emballage ou un textile.

8. Substance selon la définition de la revendication 1, destinée à une utilisation en tant que médicament.

9. Agent contenant en mélange avec des constituants usuels au moins un composé selon la revendication 1.

10. Agent selon la revendication 9, choisi parmi :
a) les agents pharmaceutiques, tels que les agents anti-tumoraux, les agents pour le traitement de maladies de la vessie, les analgésiques ;
b) les produits alimentaires, tels que les glaces, les mousses, les crèmes, les boissons, les confiseries ;
c) les agents pour l'hygiène buccale, tels que les dentifrices, les bains de bouche, les gommes à mâcher,
d) les agents de soin de la peau ou des cheveux, tels que les crèmes solaires, les crèmes pour les coups de soleil, les lotions, les shampoings, les pansements,
e) les agents répulsifs contre les insectes, les insecticides.

11. Produits textiles, tels que des chemises, des pantalons, des chaussettes, des serviettes, traités avec au moins un composé selon la revendication 1.

12. Matériau d'emballage, qui est associé avec au moins un composé selon la revendication 1.
